# EUROPEAN PATENT SPECIFICATION

(11) **EP 4 252 740 B1**
(45) Date of publication and mention of the grant of the patent: **06.08.2025**
(21) Application number: 22908812.5
(22) Date of filing: 18.11.2022
(51) Int. Cl.: A61K 9/16, A61K 31/436, A61M 37/00

(54) **MICRONEEDLE INCLUDING SURFACE-MODIFIED MICROSPHERES AND MANUFACTURING METHOD THEREFOR**
MIKRONADEL MIT OBERFLÄCHENMODIFIZIERTEN MIKROKUGELN UND HERSTELLUNGSVERFAHREN DAFÜR
MICRO-AIGUILLE COMPRENANT DES MICROSPHÈRES MODIFIÉES EN SURFACE ET SON PROCÉDÉ DE FABRICATION

(30) Priority: 31.12.2021 KR 20210194179; 31.10.2022 KR 20220143143
(43) Date of publication of application: 04.10.2023
(73) Proprietor: Small'Lab Co., Ltd., Daejeon 34-027 (KR)
(72) Inventor: JOO, So Kyoung, Bundang-gu, Seongnam-si Gyeonggi-do 13490 (KR); KIM, Byeong Su, Yuseong-gu Daejeon 34011 (KR); LEE, Jeong Gyu, Yuseong-gu Daejeon 34049 (KR); PARK, Chun Woong, Gounseo 2-gil Sejong 30063 (KR); HAN, Chang Soo, Heungdeok-gu, Cheongju-si Chungcheongbuk-do 28160 (KR); CHOI, Jae Cheol, Heungdeok-gu, Cheongju-si Chungcheongbuk-do 28222 (KR)
(74) Representative: Ter Meer Steinmeister & Partner
(86) International application number: PCT/KR2022/018353
(87) International publication number: WO 2023/128280

(56) References cited:
- CN-A- 105 998 463
- KR-A- 20160 122 051
- KR-A- 20190 080 549
- KR-B1- 101 549 086
- KR-B1- 101 549 086
- KR-B1- 101 746 024
- US-A1- 2011 137 254
- US-A1- 2021 178 138
- PARK J.-H. ET AL.: "Polymer particle-based micromolding to fabricate novel microstructures", vol. 9, no. 2, 2 April 2007 (2007-04-02), New York, pages 223 - 234, XP093112655, ISSN: 1387-2176, Retrieved from the Internet <URL:https://link.springer.com/content/pdf/10.1007/s10544-006-9024-4.pdf> DOI: 10.1007/s10544-006-9024-4

## Description

### Technical Field

The present invention relates to microneedles comprising surface-modified microspheres, and a method of preparing the same, and more particularly, to microneedles comprising surface-modified microspheres which exhibit improved residence time in the body and superior sustained-release effect, and are suitable for the treatment of diseases requiring continuous drug administration due, and a method of preparing the same.

### Background Art

The delivery of drugs through the skin, also known as transdermal drug delivery, is widely used in various fields and forms due to its convenience. Transdermal drug delivery is primarily used to deliver drugs through the skin into the systemic circulation. However, it is also utilized for delivering drugs, for example, drugs for atopic dermatitis, acne treatment, or skin disease treatment, within the skin itself. Despite such convenience and functionality, since there are many difficulties in delivering drugs through the skin due to its structure barriers, it is not easy to develop drugs passing through the skin. The cornified layer of the skin (stratum corneum) includes a brick structure consisting of keratin-rich keratinocytes and a mortar structure that fills the space between these keratinocytes with lipids such as ceramides, fatty acids, or wax. This structure acts as a barrier, resulting in very low permeability to substances. Only small substances with a low molecular weight of 500 Da or less may be delivered into the skin by diffusion, and only substances with excellent lipophilicity can permeate the skin.

To overcome the above problem, new systems such as microneedles are being developed, and microneedles can be applied in the form of a patch without any accessory equipment, making them convenient for daily use. Among the systems, a microneedle patch comprising multiple microneedles attached thereto, and these microneedles create small punctures in the skin surface to deliver drugs.

Recently, soluble microneedles based on a biodegradable polymer have been developed. After these microneedles penetrate into the skin and are biodegraded in the body, active ingredients are eluted into the skin (Korean Patent No. 10-2234446). However, the active ingredients permeating into the skin by the soluble microneedles may be escaped from subcutaneous tissue due to skin elasticity, resulting in reduced residence time in the body. Sustained-release drugs in which it is necessary for an active ingredient to exhibit its effectiveness for a long time particularly need higher residence time in the body.

Accordingly, there is a demand for the development of microneedles with improved residence time in the body and superior sustained-release effect, which are suitable for treatment of diseases requiring continuous drug administration.

KR 101 549 086 Bl presents a microneedle and microneedle patch.

PARK J.-H. ET AL.: "Polymer particle-based micromolding to fabricate novel microstructures", BIOMEDICAL MICRODEVICES, vol. 9, no. 2, 2 April 2007 (2007-04-02), pages 223-234, XP093112655, New York, ISSN: 1387-2176, DOI: 10.1007/S10544-006-9024-4.

US 2021/178138 Al presents an implantable sustained-release microneedle patch and a preparation method therefor.

CN 105 998 463 A presents a microneedle transdermal patch for treating osteoarthritis and preparation method thereof.

### Disclosure

### Technical Problems

Therefore, as a result of continuous research to meet the needs of the related art, the present inventors confirmed that microneedles prepared by using a surface-modified microsphere containing a drug have surprisingly improved residence time in the body and superior sustained-release effect, so they are suitable for use in the treatment of diseases requiring continuous drug administration, and successfully completed the present invention.

Accordingly, the object of the present invention is to provide microneedles comprising surface-modified microspheres.

Another object of the present invention is to provide a method of manufacturing microneedles comprising surface-modified microspheres.

Another object of the present invention is to provide a transdermal microneedle patch, comprising the microneedles.

### Technical Solutions

To achieve the objects of the present invention, the present invention provides microneedles comprising surface-modified microspheres containing a drug. One or more objects are achieved by the invention set out by the features of the independent claim.

The "surface-modified" microsphere used herein refers to a microsphere with a surface in which grooves such as a dimple structure which can be seen in a golf ball, wrinkles, etc., are formed.

The "microsphere" used herein is a biodegradable microsphere that serve as a carrier for delivering drugs or other substances into the body. The biodegradable microsphere has a predetermined period of degradation and disappearance in the body based on the degradation mechanism and rate of its main component, a biodegradable polymer. The release of the encapsulated drug within the microsphere occurs over a certain period of time depending on the biodegradation rate of the polymer.

The average size of the microsphere is not specifically limited, but it is desirable for it to be of a size suitable for encapsulation into microneedles, approximately 50 µm or less, and preferably, 10 µm or less.

In the present invention, the surface-modified microsphere may be formed in a single emulsion type, as an oil-in-water (O/W) type emulsion.

In the present invention, as a method of preparing microspheres, is used a solvent evaporation method, which comprises evaporating an organic solvent used in the preparation of microspheres and performing curing. In addition, a spray drying method and a sonication method may be used.

When preparing biodegradable microspheres using the solvent evaporation method, in the case of oil-in water (O/W) microspheres, which is a single emulsion, a non-polar organic solvent that is immiscible with water may be used as an internal oil phase. A biodegradable polymer and a drug can be simultaneously dissolved in the non-polar organic solvent, and this mixture can be rapidly dispersed in a water phase containing a surfactant to prepare the microspheres. Specifically, in the present invention, the microspheres are prepared by a method, which comprises i) preparing an oil phase by dissolving a drug and a surfactant-acting biodegradable polymer in an organic solvent; ii) forming an O/W emulsion by mixing the oil phase with a water phase in which a water-soluble polymer is dissolved; and iii) obtaining surface-modified microspheres by evaporating the solvent from the O/W emulsion.

The "drug" used herein is tacrolimus.

Tacrolimus has a chemical structure represented by Formula 1 and is used for the treatment of severe atopic dermatitis by inhibiting calcineurin and suppressing the production of an inflammatory mediator such as IL-2:

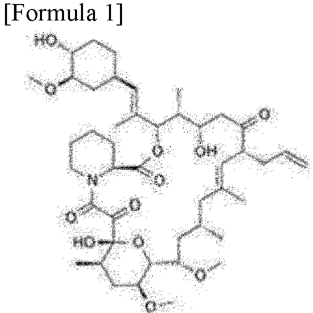

In the present invention, the surfactant-acting biodegradable polymer may be naturally biodegraded in the body and thus excreted outside the body. In addition, the surfactant-acting biodegradable polymer has the function of a surfactant, which can emulsify a water-insoluble drug. The surfactant-acting biodegradable polymer can be derived from natural sources or synthetically prepared. The surfactant-acting biodegradable polymer comprises poly(lactate-co-glycolate) (PLGA). In the present invention, PLGA is used. The surfactant-acting biodegradable polymer may have a weight average molecular weight of 5,000 to 1,000,000.

The drug and the surfactant-acting biodegradable polymer may be comprised, but are not particularly limited to, in a weight ratio of 0.1 to 10 : 10, 0.5 to 7 : 10, 3 to 7 : 10, or preferably, 4 to 6 : 10. In the above range, a surface with desired grooves (dimple structure) may be effectively formed on the microspheres.

The organic solvent may be, but is not particularly limited to, dichloromethane, methanol, ethanol, chloroform, hexane, ethyl acetate, and a mixture thereof, and preferably, dichloromethane. To the mixture of the drug and the surfactant biodegradable polymer, the organic solvent may be mixed in a mixing ratio of 1:10 to 1:30 (w/v). In the above range, it is possible to effectively form a surface with desired grooves (dimple structure) on the microspheres.

In the above step ii), an oil phase and a water phase are mixed. The water phase may comprise a dissolved water-soluble polymer, and the water-soluble polymer may be added for the stabilization of the emulsion in the emulsion-solvent evaporation process (step iii)).

In the absence of the water-soluble polymer, the process may result in uneven forms such as the aggregation of emulsions or fibrous structures. The addition of the water-soluble polymer may contribute to prevention of such a result. After the emulsion-solvent evaporation process, the water-soluble polymer may be removed through a washing step. As the water-soluble polymer, polyvinyl acetate (PVA) is used. The water-soluble polymer may have a weight average molecular weight of 1,000 to 50,000,000. In the present invention, PVA was used. The water-soluble polymer may be comprised at 0.01 to 1 wt%, and preferably, 0.1 to 0.5 wt% in the water phase. When the water-soluble polymer is used at the concentration of the above condition, the emulsion is effectively formed.

The mixing is performed using mechanical stirring means such as a homogenizer or ultrasonic waves within a range of 5,000 to 12,000 rpm (1st shear), and most preferably 12,000 rpm. Mixing in the above range enables the effective formation of desired grooves (dimple structure) on the surface of the microspheres.

The evaporation in the above step iii) is the procedure of evaporating an oil-phase organic solvent formed in the water phase, which is a continuous phase. The evaporation is performed while stirring, and the stirring may comprise mechanical stirring. Here, a stirring speed is in the range of 800 to 1,000 rpm (2^{nd} shear), and is most preferably 1,000 rpm. Stirring within this range allows for proper control of the evaporation rate, enabling the formation of desired grooves (dimple structure) on the surface of the microspheres.

Through evaporation, microspheres are formed and exist dispersed in a continuous phase of water. They can be easily collected by simple filtration. Depending on the requirements, additional purification processes such as washing to remove impurities, centrifugation, and drying may be carried out.

In the present invention, to deliver surface-modified microspheres encapsulating a drug into the skin, a material for the microneedles may be soluble so that it can be disintegrated by moisture in the skin and may be biocompatible to be absorbed or decomposed without side effects in the body. And the material preferably has sufficient strength to perforate the skin after being manufactured as microneedles.

The microneedles of the present invention are water-soluble, that is, soluble in body fluids in the skin.

As a soluble material for forming microneedles of the present invention, one or more biocompatible materials selected from the group consisting of alginic acid, hyaluronic acid, dextran (sulphate), cellulose, polyvinylpyrrolidone (PVP), polyethylene glycol (PEG), polyvinyl alcohol (PVA), sodium carboxymethylcellulose, a polyalcohol, cyclodextrin, dextrin, trehalose, sucrose, lactose, mannitol, and xylitol; and mixtures thereof may be used. In one embodiment of the present invention, a mixture of alginic acid and trehalose is used as the soluble material.

The microneedles of the present invention may further comprise a plasticizer, a surfactant, and a preservative.

As a plasticizer, for example, polyols such as ethylene glycol, propylene glycol, dipropylene glycol, butylene glycol, glycerin, etc. may be used alone or in combination, but the present invention is not limited thereto. As a surfactant, for example, PEG-8 glyceryl isostearate, PEG-10 glyceryl isostearate, PEG-15 glyceryl isostearate, PEG-20 hydrogenated castor oil, PEG-30 hydrogenated castor oil, PEG-40 hydrogenated castor oil, PEG-60 hydrogenated castor oil, PEG-80 hydrogenated castor oil, and ceteareth-12 may be used alone or in combination, but the present invention is not limited thereto. As a preservative, for example, methyl parahydroxybenzoate, ethyl parahydroxybenzoate, paraoxybenzoic acid, (iso)propylparaoxybenzoic acid, (iso)butylchlorobutanol (chlorobutol), benzalkonium chloride, benzethonium chloride, phenol (p-form), cresol, chlorocresol, dehydroacetic acid, sodium dehydroacetate, sorbic acid, potassium sorbate, sodium sorbate, benzoic acid, and sodium benzoate may be used alone or in combination, but the present invention is not limited thereto.

The needle part of the microneedles according to the present invention may be a conical, pyramidal, spear-shaped, truncated, wedge-shaped, or blade-shaped, or the like, all of which have commonly penetrate the skin. In one embodiment of the present invention, microneedles with a pyramid-like needle part, which is structurally stable, are selected.

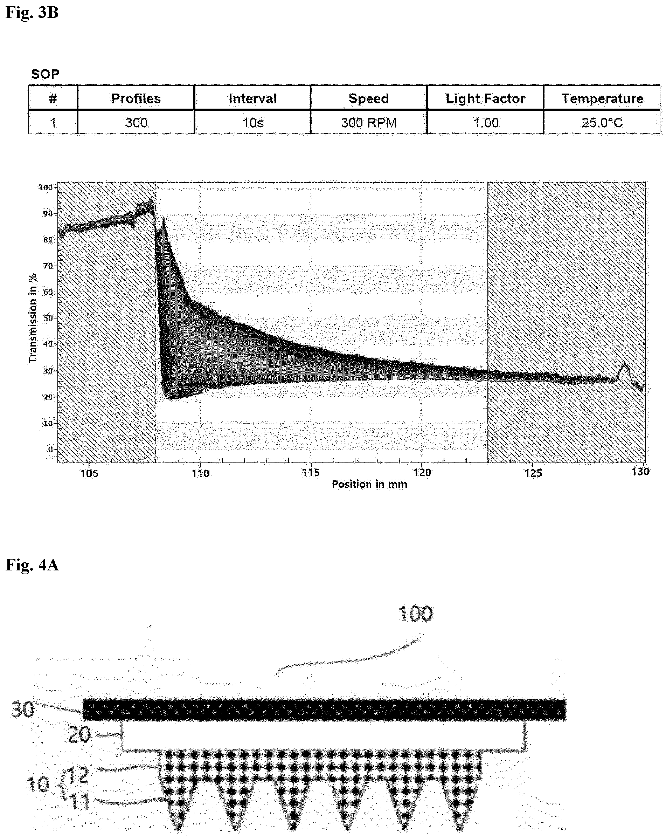

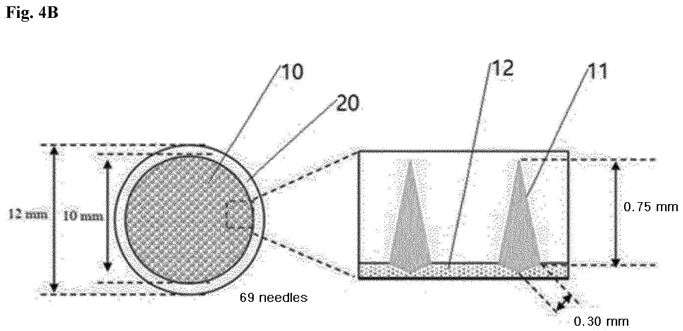

The structures of microneedles 10 and a microneedle patch 100 according to the present invention are shown in FIGS. 4A and 4B. The microneedles 10 of the present invention may comprise a needle part 11 and a matrix layer 12. The needle part 11 has a shape that is easy to penetrate the skin as defined above. The length of the needle part 11 may be 500 to 1,000 µm, and preferably, 750 µm. The thickness of the matrix layer 12 may be 0.1 to 1 mm, and preferably, 0.1 to 0.3 mm. The needle part and the matrix layer encapsulate (contain) a surface-modified microsphere encapsulating a drug. The microneedle patch 100 of the present invention is formed by laminating an adhesive layer 20 on one side of the matrix layer 12, allowing the microneedle patch to be adhered to the skin. In the microneedle patch 100, the adhesive layer part excluding the portion in contact with the microneedles 10 accounts for 50% or less of the entire area of the adhesive layer. The microneedle patch 100 may also comprise a protective film 30 on the adhesive layer.

The present disclosure provides a method of preparing the microneedles.

The preparing method comprises
a) forming a first solution by dissolving a soluble material in water,
b) preparing surface-modified microspheres containing (encapsulating) a drug,
c) preparing a mixed solution by mixing and homogenizing the first solution and the surface-modified microspheres,
d) filling an engraved microneedle mold with the mixed solution, and
e) drying the mixed solution filled in the mold and separating the resulting product from the mold.

In the manufacturing method of the present disclosure, the soluble material, the drug, the surface-modified microspheres are defined as described above.

In step a), the first solution may further comprise a plasticizer, a surfactant, and a preservative. The plasticizer, the surfactant, and the preservative, etc., are defined as described above.

A method of preparing the surface-modified microsphere in step b) is defined as described above.

In step c), with respect to 100 parts by weight of the first solution, 0.1 to 20 parts by weight of the surface-modified microsphere is mixed.

In the step of preparing the mixed solution, it is important to ensure that the surface-modified microparticles containing a drug are uniformly distributed within the microneedles. Accordingly, after mixing the first solution and the surface-modified microspheres, vigorous homogenization such as vortexing are applied to achieve a stable and homogeneous dispersion of the microparticles in the mixed solution.

Conditions such as a temperature used in manufacturing the microneedles are not particularly limited as long as the soluble material or surface-modified microspheres can be sufficiently dissolved or mixed without decomposition or deformation.

In step d), the filling of a microneedle mold with the mixed solution may use a method of allowing the mixed solution to stand after applying it, a method of injecting the mixed solution using a centrifuge, a method of injecting the mixed solution by vacuuming to eliminate internal air, or a method of injecting the mixed solution under pressure.

In step e), drying may be performed at room temperature, or may be performed at room temperature to 80 °C using hot air dryer, but the present invention is not limited thereto.

According to still another object of the present invention, the present invention provides a transdermal microneedle patch, which comprises the above-described microneedles comprising surface-modified microspheres containing a drug, or microneedles comprising surface-modified microspheres containing a drug, prepared by the above-described preparing method.

When the drug is tacrolimus, the transdermal microneedle patch may be used for treating and alleviating atopic dermatitis and acne.

### Advantageous Effect

Microneedles comprising surface-modified microspheres containing a drug according to the present invention exhibit improved residence time in the body and a superior sustained-release effects, and are therefore useful for treatment of conditions and diseases requiring continuous drug administration

### Description of Drawings

FIGS. 1A and 1B are electron micrographs of microspheres produced in Preparation Example 1.
FIG. 2 is a set of electron micrographs of tacrolimus-containing microspheres whose surface is modified into a dimple structure of Formulation 7, prepared for the purpose of reproducibility verification.
FIG. 3A shows the results of measuring, using LUMiSizer, the dispersion stability of tacrolimus-containing microspheres whose surface is modified into a dimple structure according to the present invention.
FIG. 3B is the results of measuring, using LUMiSizer, the dispersion stability of conventional microspheres containing tacrolimus with a conventional smooth surface.
FIGS. 4A and 4B are schematic diagrams of examples of microneedles and a microneedle patch according to the present invention.
FIG. 5 shows electron micrographs of microneedles according to the present invention.
FIG. 6 shows the results of analysis of residual amount of tacrolimus in dermis over time after a microneedle patch according to the present invention is attached to rat skin.

### Examples of the Invention

Hereinafter, the configuration and effects of the present invention will be described in further detail with reference to exemplary examples to better describe the present invention. However, the following examples are merely exemplified to more clearly describe the present invention, and the scope of the present invention is not limited by the following examples.

### Preparation Example 1: Preparation of surface-modified microsphere containing tacrolimus

Tacrolimus-containing microspheres were prepared using solvent evaporation method for an oil-in-water (O/W) emulsion. Conditions for a formulation composition, an oil phase, a water phase, a solvent composition, a homogenizer, and a mechanical stirrer are shown in Table 1 below.

Specifically, tacrolimus 120 mg and PLGA 503H (Evonik Ltd., Germany) 300 mg were dissolved in dichloromethane at the amounts indicated in Tables 1 and 2 to prepare the oil phase. Then an O/W emulsion was formed by mixing the oil phase with a water phase of a 0.5 to 1% polyvinyl alcohol (PVA 500, OCI Company, Ltd., Korea) solution using a homogenizer under the conditions shown in Table 1 for 2 minutes. The O/W emulsion was stirred using a mechanical stirrer under the conditions shown in Table 1 for 3 hours or more to evaporate an organic solvent, thereby forming microspheres. To remove the remaining PVA, and drug particles not encapsulated by the polymer, centrifugation was performed at X 820g for 5 minutes, followed by washing with distilled water three times and then subjected to freeze-drying for 2 days to obtain a powder-type particles.

**[Table 1]**

| Formulation | No. | 1 | 2 | 3 | 4 | 5 | 6 | 7 | 8 | 9 |
|---|---|---|---|---|---|---|---|---|---|---|
| | Tacrolimus (mg) | 120 | 120 | 120 | 120 | 120 | 120 | 120 | 120 | 120 |
| | PLGA 503H (mg) | 300 | 300 | 300 | 300 | 300 | 300 | 300 | 300 | 300 |
| Oil phase | DCM (mL) | 15 | 15 | 15 | 10 | 10 | 10 | 10 | 10 | 10 |
| Water phase | PVA solution (w/v, %) | 0.5%, 600 mL | 0.5%, 600 mL | 0.5%, 600 mL | 0.5%, 600 mL | 0.5%, 600 mL | 0.5% 600 mL | 0.5% 600 mL | 0.5%, 600 mL | 0.5%, 600 mL |
| 1^{st} Shear (Homogeniz er) | Homogenizer (rpm) | 10,00 0 | 10,00 0 | 5,000 | 10,00 0 | 12,00 0 | 15,00 0 | 12,00 0 | 12,00 0 | 12,00 0 |
| 2nd Shear (Mechanical stirrer) | Stirring rate (rpm) | 1000 | 500 | 1000 | 1000 | 500 | 800 | 1000 | 100 | 500 |

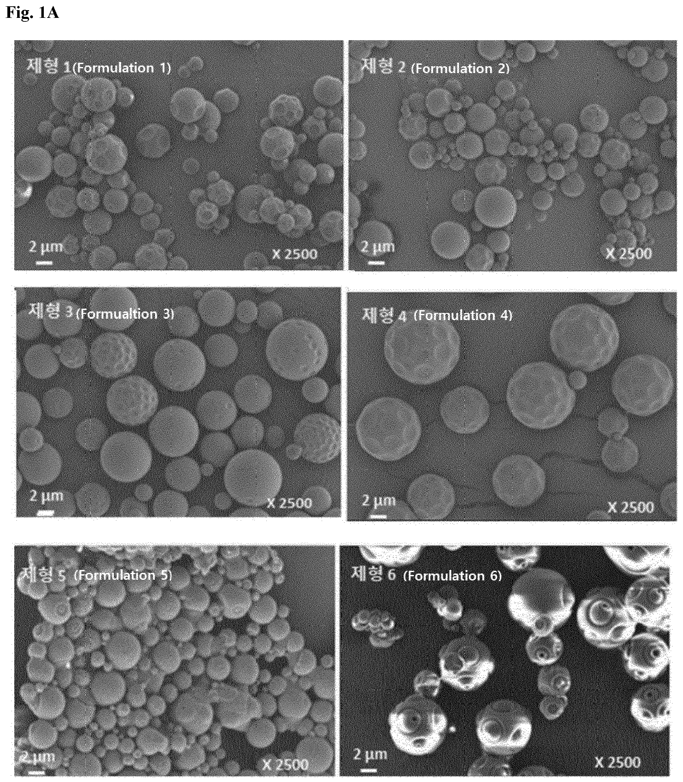

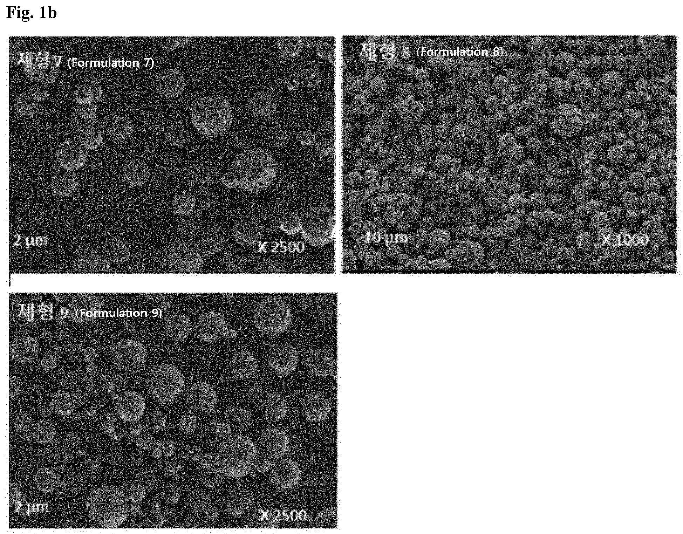

The distribution of particle sizes of prepared tacrolimus-containing microspheres of each formulation was measured. The results are shown in Table 2, and the electron micrographs of the microspheres are shown in FIGS. 1A and 1B.

**[Table 2]**

| Formulation | 1 | 2 | 3 | 4 | 5 | 6 | 7 | 8 | 9 |
|---|---|---|---|---|---|---|---|---|---|
| Dv10 (µm) | 1.13 | 1.2 | 1.14 | 1 | 1.15 | 1.69 | 1.21 | 0.196 | 0.191 |
| Dv50 (µm) | 3.06 | 3.15 | 7.06 | 6.77 | 4.62 | 6.84 | 5.96 | 5.81 | 4.34 |
| Dv90 (µm) | 6.46 | 7.39 | 14.8 | 12.6 | 10.4 | 11.11 | 10.6 | 20.5 | 13.4 |
| Span | 1.741 | 1.96 | 1.94 | 1.71 | 2.00 | 1.38 | 1.57 | 3.50 | 2.86 |
| Yield (%) | 69 | 64 | 55.4 | 69.9 | 38.5 | 51.0 | 59.8 | 37.6 | 45.7 |

Referring to FIGS. 1A and 1B, in Formulations 4 and 7, tacrolimus-containing microspheres whose surface was modified into a dimple structure were identified, and in Formulations 1, 2, 3, 5, 6, 8, and 9, most of the microspheres have a conventional smooth surface.

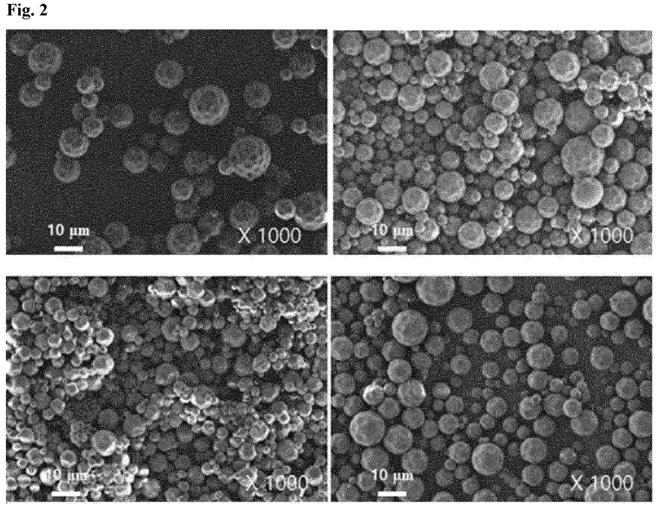

For Formulation 7, reproducibility was checked, and as shown in FIG. 2, tacrolimus-containing microspheres whose surface was uniformly modified into a dimple structure were obtained in all four trials. The results of analyzing of Formulation 7 collected from the four trials, are shown in Table 3.

**[Table 3]**

| Formulation 7 | |
|---|---|
| Yield (%) | Yield (%) |
| Entrapment efficiency (%) | 18.77 ± 0.79 |
| Dv10 (µm) | 0.307 |
| Dv50 (µm) | 8.03 |
| Dv90 (µm) | 20.6 |
| Span | 2.531 |

### <Optimization of conditions for preparing surface-modified microspheres>

To establish the optimal conditions for preparing tacrolimus-containing microspheres whose surface is modified into a dimple structure, microspheres were further prepared by modifying the preparation conditions for the formulations shown in Table 1. In Formulation 1, when the PVA content in the water phase was increased to 1.5%, but it had no significant effect. When the evaporation step was carried out at an increased stirring speed (2nd Shear) of 1,000 rpm in Formulation 2, surface-modified microspheres with a dimple structure were obtained. In Formulation 3, reducing the amount of an oil-phase solvent (DCM) to 10 mL resulted in the production of surface-modified microspheres with a dimple structure. In Formulation 5, Increasing the stirring speed (2^{nd} shear) upon evaporation to 1,000 rpm also led to the production of surface-modified microspheres with a dimple structure. In Formulation 6, lowering the homogenizer mixing speed (1^{st} shear) to 12,000 rpm resulted in the formation of surface-modified microspheres with a dimple structure. In Formulations 8 and 9, increasing the stirring speed (2^{nd} shear) upon evaporation to 800 to 1,000 rpm yielded surface-modified microspheres with a dimple structure.

When summarizing the above preparation experiment results, the desirable conditions for the production of surface-modified microspheres with a dimple structure are as follows: for the mixing of an oil phase and a water phase, a range of 5,000 to 15,000 rpm (1st shear) is preferred, with the most desirable speed being 12,000 rpm. During the solvent evaporation step, stirring is desirably carried out within the range of 800 to 1,000 rpm (2nd shear), with the most desirable speed being 1,000 rpm. Regarding the addition of an organic solvent to the mixture of a drug and a surfactant-acting biodegradable polymer in the oil phase, the solvent is preferably added at a ratio of 1:10 to 1:30 (w/v).

### Experimental Example 1: Analysis of dispersion stability of surface-modified microsphere

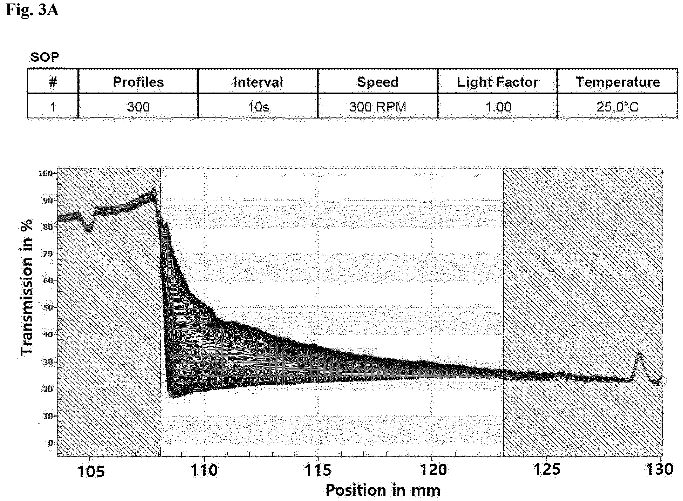

The dispersion stability of surface-modified (dimple-structure) microspheres containing tacrolimus (Formulation 7) and microspheres with a smooth surface (Formulation 9), which were prepared in Preparation Example 1, was measured using LUMiSizer, and the results are presented in FIGS. 3A and 3B, respectively, along with Table 4:

**[Table 4]**

| Microspheres | Instability Index | Mean RCA in g | Time in s |
|---|---|---|---|
| Formulation 7 | 0.234 | 11.63 | 2,991 |
| Formulation 9 | 0.261 | 11.62 | 3,002 |

It can be confirmed that the surface-modified (dimple-structure) microspheres according to the present invention have a low instability index value, indicating a high dispersion stability.

### Preparation Example 2: Manufacture of microneedles comprising surface-modified microspheres

Microneedles of Example 1 comprising the surface-modified (dimple-structure) microspheres containing tacrolimus (Formulation 7) prepared in Preparation Example 1 were prepared. Also microneedles of Comparative Example 1 comprising the microsphere with a smooth surface (Formulation 9) prepared in Preparation Example 1 were prepared.

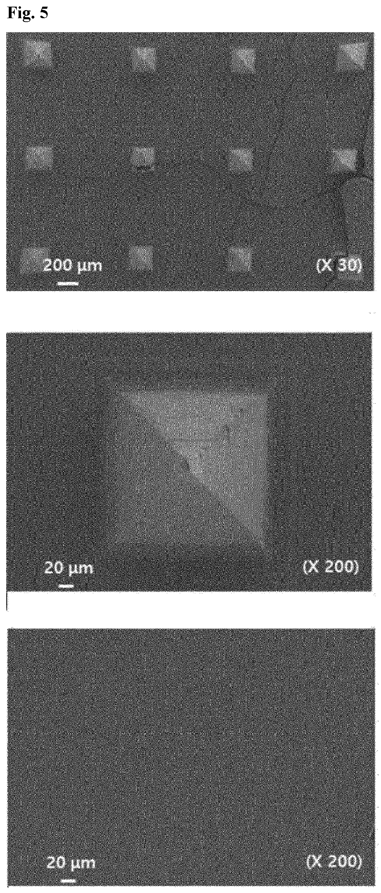

Specifically, a first solution was prepared by dissolving 1.0 g of sodium alginate (SUNFINE GLOBAL) and 1.0 g of trehalose (SUNFINE GLOBAL) in 33 g of H₂O. Then 9.9 g of the first solution was mixed with 0.1 g of the surface-modified microspheres of Formulation 7 prepared in Preparation Example 1, or the microsphere with a smooth surface of Formulation 9 prepared in Preparation Example 1, respectively, under vortexing for 5 minutes or more to disperse for homogenization. The resulting mixture was then filled into a pyramid-like engraved silicone mold with a depth of 750 µm. The mold was placed in a desiccator, subjected to a vacuum of -0.04 Mpa for 30 minutes, and dried in a hot air dryer at 50 °C for 1.5 hours. The dried microneedles were collected using adhesive tape, and the edges were rounded by cutting with scissors to match the patch shape (FIG. 4B). The electron micrographs of the completed microneedles were taken and shown in FIG. 5.

As shown in FIG. 5, it can be observed that the microspheres are well-formed.

In addition, the strength of the completed microneedles of Example 1 was evaluated under the conditions of Table 5 using a texture analyzer, and the results are presented in Table 6:

**[Table 5]**

| Measurement conditions (TA.XT Plus Texture analyzer) | |
|---|---|
| Mode | Compression |
| Pre-Test Speed | 1.00mm/sec |
| Test Speed | 0.1mm/sec |
| Post-Test Speed | 10.00 mm/sec |
| Distance | 0.800 mm |
| Trigger Force | 0.49 |

**[Table 6]**

| | Strength per Needle (N/number) | | | |
|---|---|---|---|---|
| | No. | Strength (N) | Average | STD |
| Example 1 | 1 | 2.01 | 2.02 | 0.07 |
| | 2 | 1.95 | | |
| | 3 | 2.09 | | |

As indicated in Table 6, the average strength of the microneedles of Example 1 is 2.02, which is considered to be a sufficient strength for skin penetration.

### Experimental Example 2: Analysis of residence time in the subcutaneous body of drug

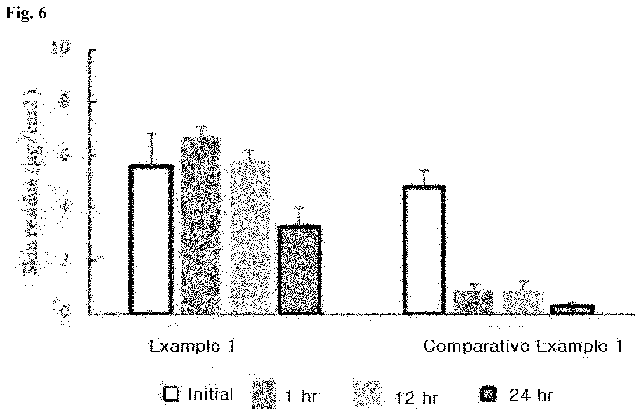

The hairless dorsal skin tissue of a 6-week-old male SD rat was collected and used in an *in-vitro* Franz cell permeation test. Each of the microneedle patches of Example 1 and Comparative Example 1, manufactured in Preparation Example 2, was attached to the dorsal skin for 0.5 minutes and then removed. Samples from the dorsal skin were taken at 0, 1, 12, or 24 hours. The surface of the sampled skins was wiped with an alcohol swab and extracted using an HPLC mobile phase. The supernatant from the resulting extracts was quantitatively analyzed using HPLC and the results are presented in Table 7 and FIG. 6.

**[Table 7]**

| Sampling Time | Example 1 | | Comparative Example 1 | |
|---|---|---|---|---|
| | Average (µg/cm² ) | STDEV | Average (µg/cm²) | STDEV |
| Initial | 5.6 | 1.2 | 4.8 | 0.6 |
| 1 hr | 6.7 | 0.4 | 0.9 | 0.2 |
| 12 hr | 5.8 | 0.4 | 0.9 | 0.3 |
| 24 hr | 3.3 | 0.7 | 0.3 | 0.1 |

As shown in FIG. 6 and Table 7, when using the microneedle patch comprising the microspheres having a dimple structure of Example 1, the amount of residual tacrolimus in the subcutaneous tissue was high at 3.3 µg/cm² after 24 hours. However when using the microneedle patch comprising the microspheres with a smooth surface of Comparative Example 1, the amount of residual tacrolimus in the subcutaneous tissue was low at 0.3 µg/cm².

Injected microspheres into skin may be removed from the subcutaneous tissue due to skin elasticity. It can be observed that the microspheres with a dimple structure in Example 1, due to their surface roughness, are not detached from the subcutaneous tissue and exhibit higher residence time in the body compared to the microneedles with a smooth surface in Comparative Example 1. Accordingly, it can be seen that the microneedles comprising surface-modified microspheres according to the present invention have increased residence time in the body, ultimately achieving long-acting effect (sustained-release effect) of a drug within the microspheres.

### Description of reference numerals

10: Microneedles
11: Needle part
12: Matrix layer
20: Adhesive layer
30: Protective film
100: Microneedle patch

## Claims

1. Microneedles soluble in the skin and having improved residence time in the body, the microneedles comprising surface-modified microspheres encapsulating a drug,
wherein the surface-modified microspheres are biodegradable and have a surface on which a dimple structure is formed, wherein the microspheres are prepared by solvent evaporation method comprising:
i) preparing an oil phase by dissolving a drug and a surfactant-acting biodegradable polymer in an organic solvent;
ii) forming an O/W, oil-in-water, emulsion by mixing the oil phase with a water phase in which a water-soluble polymer is dissolved; and
iii) obtaining surface-modified microspheres by evaporating the solvent from the O/W emulsion, the evaporation performed under stirring at a speed of 800 to 1,000 rpm; and
wherein:
- the surfactant-acting biodegradable polymer is poly(lactate-co-glycolate) (PLGA);
- the water-soluble polymer is polyvinyl acetate (PVA);
- the drug is tacrolimus; and
- a soluble material for forming the microneedles is any one selected from the group consisting of alginic acid, hyaluronic acid, dextran, cellulose, polyvinylpyrrolidone (PVP), polyethylene glycol (PEG), polyvinyl alcohol (PVA), sodium carboxymethylcellulose, polyalcohol, cyclodextrin, dextrin, trehalose, sucrose, lactose, mannitol, xylitol and mixtures thereof.

2. The microneedles of claim 1, wherein the average size of the microspheres is 50 µm or less.

3. The microneedles of claim 1, wherein the mixing in step ii) is performed at a speed of 5,000 to 12,000 rpm.

4. The microneedles of claim 1, wherein the soluble material for forming microneedles is a mixture of alginic acid and trehalose.

5. The microneedles of any one of the preceding claims, wherein the shape of the needle part of the microneedles has any one of a conical, pyramidal, spear-shaped, truncated, wedge-shaped, or blade-shaped.

6. The microneedles of any one of the preceding claims, wherein the length of the needle part of the microneedles is 500 to 1,000 µm.

7. A transdermal microneedle patch comprising the microneedles comprising surface-modified microspheres encapsulating containing a drug according to any one of the preceding claims.

## Patentansprüche

1. Mikronadeln, die in der Haut auflösbar sind und eine verbesserte Verweilzeit im Körper aufweisen, wobei die Mikronadeln oberflächenmodifizierte Mikrokugeln, die ein Medikament einkapseln, umfassen,
wobei die oberflächenmodifizierten Mikrokugeln biologisch abbaubar sind und eine Oberfläche aufweisen, auf der eine Vertiefungsstruktur gebildet ist, wobei die Mikrokugeln durch ein Lösungsmittelverdampfungsverfahren hergestellt werden, das Folgendes umfasst:
i) Herstellen einer Ölphase durch Lösen eines Medikaments und eines oberflächenaktiv wirkenden, biologisch abbaubaren Polymers in einem organischen Lösungsmittel;
ii) Bilden einer Ö/W-Emulsion, Öl-in-Wasser-Emulsion, durch Mischen der Ölphase mit einer Wasserphase, in der ein wasserlöslicher Polymer gelöst ist; und
iii) Erhalten der oberflächenmodifizierten Mikrokugeln durch Verdampfen des Lösungsmittels aus der Ö/W-Emulsion, wobei das Verdampfen unter Rühren mit einer Drehzahl im Bereich von 800 bis 1000 min⁻¹ durchgeführt wird; und
wobei:
- der oberflächenaktiv wirkende, biologisch abbaubare Polymer Poly(lactid-co-glycolid) (PLGA) ist;
- der wasserlösliche Polymer Polyvinylacetat (PVA) ist;
- das Medikament Tacrolismus ist; und
- ein auflösbares Material zum Bilden der Mikronadeln irgendeines ist, das aus der Gruppe ausgewählt wird, die aus Alginsäure, Hyaluronsäure, Dextran, Cellulose, Polyvinylpyrrolidon (PVP), Polyethylenglykol (PEG), Polyvinyl-Alkohol (PVA), Natriumcarboxymethylcellulose, Polyalkohol, Cyclodextrin, Dextrin, Trehalose, Saccharose, Lactose, Mannitol, Xylitol und Gemischen davon besteht.

2. Mikronadeln nach Anspruch 1, wobei die durchschnittliche Größe der Mikrokugeln 50 µm oder kleiner ist.

3. Mikronadeln nach Anspruch 1, wobei das Mischen in Schritt ii) mit einer Drehzahl im Bereich von 5000 bis 12000 min⁻¹ durchgeführt wird.

4. Mikronadeln nach Anspruch 1, wobei das auflösbare Material zum Bilden der Mikronadeln eine Gemisch aus Alginsäure und Trehalose ist.

5. Mikronadeln nach einem der vorhergehenden Ansprüche, wobei die Form des Nadelabschnitts der Mikronadeln irgendeine Form von konisch, pyramidenförmig, spießförmig, kegelstumpfförmig, keilförmig oder klingenförmig aufweist.

6. Mikronadeln nach einem der vorhergehenden Ansprüche, wobei die Länge des Nadelabschnitts der Mikronadeln im Bereich von 500 bis 1000 µm liegt.

7. Hautdurchdringendes Mikronadelpflaster, das die Mikronadeln, die die oberflächenmodifizierten Mikrokugeln, die ein Medikament einkapselnd enthalten, umfassen, nach einem der vorhergehenden Ansprüche umfasst.

## Revendications

1. Micro-aiguilles (10) solubles dans la peau et ayant un temps de séjour amélioré dans le corps, les micro-aiguilles (10) comportant des microsphères modifiées en surface encapsulant un médicament,
dans lesquelles les microsphères modifiées en surface sont biodégradables et ont une surface sur laquelle une structure bosselée est formée, dans lesquelles les microsphères sont préparées par un procédé d'évaporation de solvant comportant de :
i) préparer une phase huileuse en dissolvant un médicament et un polymère biodégradable agissant comme tensioactif dans un solvant organique ;
ii) former une émulsion huile dans eau, O/W, en mélangeant la phase huileuse avec une phase aqueuse dans laquelle un polymère soluble dans l'eau est dissous ; et
iii) obtenir des microsphères modifiées en surface par évaporation du solvant à partir de l'émulsion O/W, l'évaporation étant réalisée sous agitation à une vitesse de 800 à 1 000 t/min ; et
dans lesquelles :
- le polymère biodégradable agissant comme tensioactif est le poly(lactate-co-glycolate) (PLGA) ;
- le polymère soluble dans l'eau est le polyacétate de vinyle (PVA) ;
- le médicament est le tacrolimus ; et
- un matériau soluble pour former les micro-aiguilles est un matériau quelconque choisi parmi le groupe constitué d'acide alginique, d'acide hyaluronique, de dextrane, de cellulose, de polyvinylpyrrolidone (PVP), de polyéthylène glycol (PEG), d'alcool polyvinylique (PVA), de carboxyméthylcellulose sodique, d'un polyalcool, de cyclodextrine, de dextrine, de tréhalose, de saccharose, de lactose, de mannitol, de xylitol et de mélanges de ceux-ci.

2. Micro-aiguilles selon la revendication 1, dans lesquelles la taille moyenne des microsphères est de 50 µm ou moins.

3. Micro-aiguilles selon la revendication 1, dans lesquelles le mélange à l'étape ii) est réalisé à une vitesse de 5 000 à 12 000 t/min.

4. Micro-aiguilles selon la revendication 1, dans lesquelles le matériau soluble pour former des micro-aiguilles est un mélange d'acide alginique et de tréhalose.

5. Micro-aiguilles selon l'une quelconque des revendications précédentes, dans lesquelles la forme de la partie d'aiguille des micro-aiguilles a une forme quelconque parmi une forme conique, pyramidale, en pointe, tronquée, en coin ou en lame.

6. Micro-aiguilles selon l'une quelconque des revendications précédentes, dans lesquelles la longueur de la partie d'aiguille des micro-aiguilles est de 500 à 1 000 µm.

7. Timbre transdermique à micro-aiguilles comportant les micro-aiguilles comportant des microsphères modifiées en surface encapsulant contenant un médicament selon l'une quelconque des revendications précédentes.
